# EUROPÄISCHE PATENTSCHRIFT

(11) **EP 3 984 433 B1**
(45) Veröffentlichungstag und Bekanntmachung des Hinweises auf die Patenterteilung: **17.04.2024**
(21) Anmeldenummer: 21197050.4
(22) Anmeldetag: 16.09.2021
(51) Int. Cl.: A47L 9/28

(54) **REINIGUNGSROBOTER FÜR EINE GEPOLSTERTE FLÄCHE**
CLEANING ROBOT FOR CUSHIONED SURFACE
ROBOT DE NETTOYAGE POUR UNE SURFACE REMBOURRÉE

(30) Priorität: 24.09.2020 DE 102020212047
(43) Veröffentlichungstag der Anmeldung: 20.04.2022
(60) Teilanmeldung: 22166044.2
(73) Patentinhaber: BSH Hausgeräte GmbH, 81739 München (DE)
(72) Erfinder: Fremerey, Maximilian, 97633 Saal an der Saale (DE)

(56) Entgegenhaltungen:
- EP-A2- 2 478 814
- CN-B- 106 075 493
- US-A1- 2018 299 899

## Beschreibung

Die Erfindung betrifft einen Reinigungsroboter. Insbesondere betrifft die Erfindung einen Reinigungsroboter für eine gepolsterte Fläche. Ein Reinigungsroboter gemäß dem Oberbegriff des Anspruchs 1 ist z.B. aus US-A-2018299899 bekannt.

In einem Haushalt befinden sich eines oder mehrere gepolsterte Möbelstücke, wie beispielsweise ein Sofa, ein Sessel oder ein Bett. Zur Wahrung der Hygiene im Haushalt ist es üblich, gepolsterte Flächen der Möbelstücke regelmäßig zu reinigen. Bei einer üblichen Reinigung mittels Absaugens können jedoch Kleinlebewesen, die sich in einer Polsterfläche eingenistet haben, insbesondere Milben, oft nicht adäquat entfernt werden. Die sich überwiegend von Hautschuppen ernährenden Milben stellen zwar an sich keine Gefahr für einen Menschen dar, jedoch sind ihre Ausscheidungen hochgradig allergen, sodass die Gefahr besteht, dass ein Benutzer eines solchen Polstermöbels unter allergischen Beschwerden leidet.

Es wurde vorgeschlagen, Milben mittels einer starken Lampe zur Aussendung von ultraviolettem Licht zu bekämpfen. Das UV-Licht kann mittels eines autonomen Roboters beim Abfahren der Fläche ausgesandt werden. Solches kurzwellige Licht kann jedoch schädlich sein, wenn es in die Augen eines Menschen oder eines Haustiers gerät und Reizungen oder Hautirritationen bereits dann hervorrufen, wenn es über längere Zeit auf ungeschützte Haut fällt. Es wurde auch vorgeschlagen, zur Bekämpfung von Milben ein nichtthermisches Plasma zu verwenden. Dieses kann jedoch Ozon freisetzen, das bereits in geringen Konzentrationen für gesundheitlich vorbelastete Personen ein Reizklima bewirken kann. Außerdem kann eine flächendeckende Behandlung einer Oberfläche eines Polstermöbels mit ultraviolettem Licht oder mit Plasma energieaufwendig oder langwierig sein. Ein Roboter kann viel Energie zur Reinigung eines größeren Polstermöbels wie beispielsweise eines Doppelbetts erfordern, sodass er einen großen Energiespeicher benötigen kann. Ein kleinerer Energiespeicher muss unter Umständen vor Beenden der Reinigung nachgeladen werden, sodass die Reinigung insgesamt sehr lange dauern kann.

Eine der vorliegenden Erfindung zu Grunde liegende Aufgabe besteht in der Angabe einer verbesserten Technik zur autonomen Reinigung einer gepolsterten Fläche. Die Erfindung löst diese Aufgabe mittels der Gegenstände der unabhängigen Ansprüche. Unteransprüche geben bevorzugte Ausführungsformen wieder.

Nach einem ersten Aspekt der vorliegenden Erfindung umfasst ein Reinigungsroboter für eine gepolsterte Fläche eine Antriebseinrichtung; eine Steuereinrichtung zur Führung des Reinigungsroboters über die zu reinigende Fläche; eine Reinigungseinrichtung, die dazu eingerichtet ist, Milben im Bereich der Fläche zu bekämpfen; und eine Ausgabevorrichtung für ein Lockmittel für Milben. Dabei ist die Steuereinrichtung dazu eingerichtet, Lockmittel auf der Fläche auszugeben und nach einer vorbestimmten Wartezeit die Reinigungseinrichtung im Bereich des ausgegebenen Lockmittels zu aktivieren. Das Bekämpfen kann ein Aufnehmen und/oder ein Deaktivieren oder Zerstören von Milben umfassen.

Die gepolsterte Fläche kann insbesondere ein Sitz- oder Liegemöbel wie einen Sessel, eine Couch, einen Stuhl oder ein Bett umfassen. Die gepolsterte Fläche kann auch einen Boden bedecken und etwa einen Teppich oder einen Teppichboden umfassen. Die Fläche liegt bevorzugt in einem Haushalt vor, insbesondere einem Privathaushalt.

Es wurde erkannt, dass ein flächendeckendes Reinigen der Fläche vermieden werden kann, indem die Milben an eine vorbestimmte Position gelockt und dort gezielt aufgenommen bzw. bekämpft werden. Ein Energieaufwand zum Bewegen des Reinigungsroboters oder zum Betreiben der Reinigungseinrichtung kann verringert sein. Der Reinigungsroboter kann einen verkleinerten Energiespeicher aufweisen, sodass er insgesamt kleiner oder leichter konstruiert werden kann. Ein Reinigungsvorgang kann schneller abgeschlossen sein.

Das Bekämpfen kann ein Inaktivieren von Milben umfassen. Beim Inaktivieren kann eine Milbe so behandelt werden, dass ihre Schadwirkung möglichst minimiert ist. Die Schadwirkung einer Milbe kann in ihrer Verbreitung von Ausscheidungen, die allergen wirken können, insbesondere Kot, auf der Fläche bestehen. Das Inaktivieren kann eine Milbe bewegungsunfähig machen, sodass ihre Schadwirkung auf der Fläche stark eingeschränkt ist. Daneben kann das Inaktivieren die Milbe so weit schädigen, dass sie keinen nennenswerten Stoffwechsel mehr betreibt. Insbesondere kann eine Ausscheidung von Exkrementen unterbunden sein. Das Inaktivieren kann auch ein Abtöten umfassen.

Die Reinigungseinrichtung ist bevorzugt dazu eingerichtet ist, Milben von der Fläche zu entfernen. Eine inaktivierte Milbe kann sich nicht mehr aktiv auf der Fläche halten, sodass sie verbessert entfernt werden kann. Von der Fläche entfernte Milben und/oder Schmutz können in einem Behälter gesammelt werden. Dazu können Feststoffe von einer an der Fläche abgesaugten Luft abgeschieden werden, beispielsweise durch einen Tiefenfilter oder einen Zyklonfilter. Durch das vorausgehende Inaktivieren kann verhindert werden, dass Milben aus dem Behälter entkommen.

Das Entfernen von Milben kann auf einem Teil der Fläche erfolgen, auf dem zuvor Lockmittel ausgebracht wurde. In einer Ausführungsform kann das Entfernen auf einem Teil der Fläche, auf dem zuvor kein Lockmittel ausgebracht wurde, unterbleiben. Eine Einrichtung zum Ausbringen von Lockmittel kann eine vorbestimmte Bearbeitungsbreite aufweisen, und eine Einrichtung zur Bekämpfung und insbesondere Inaktivierung kann eine entsprechende Bearbeitungsbreite aufweisen. Das Entfernen der Milben kann ebenfalls auf einer entsprechenden Bearbeitungsbreite erfolgen. Durch zueinander korrespondierende Bearbeitungsbreiten kann die Fläche effizienter gereinigt werden. Ein Aufwand zur Bewegung des Reinigungsroboters über die Fläche kann minimiert sein.

Das Lockmittel ist bevorzugt dazu eingerichtet, die Milben nicht zu schädigen. Das Lockmittel kann insbesondere nicht toxisch für Milben sein. Dadurch muss nicht gewartet werden, bis eine inaktivierende Wirkung des Lockmittels eingetreten ist. Außerdem kann es einfacher sein, ein reines Lockmittel bereitzustellen, als ein Lockmittel mit schädigender Wirkung. Das Inaktivieren der Milben erfolgt bevorzugt mittels einer elektromechanischen Einrichtung, die beispielsweise ein nichtthermisches Plasma bereitstellen kann, wie unten genauer ausgeführt ist.

Die Steuereinrichtung ist bevorzugt dazu eingerichtet, eine Emission von Schall oder Vibrationen durch den Reinigungsroboter während der Wartezeit zu minimieren, idealerweise ganz zu unterdrücken. Das Anlocken von Milben kann dadurch unterstützt werden. Durch das Ausbleiben von Schallwellen oder Vibrationen, die eine Milbe dazu bringen können, sich nicht zu bewegen, sich zu verstecken, sich festzuhalten oder zu fliehen, können die Milben effektiver angelockt werden. Eine größere Anzahl Milben kann angelockt werden oder eine erforderliche Wartezeit zum Anlocken der Milben kann verkürzt sein.

Es ist besonders bevorzugt, dass die Steuereinrichtung dazu eingerichtet ist, das Lockmittel an einer vorbestimmten Stelle der gepolsterten Fläche auszugeben. Die Stelle kann insbesondere in Abhängigkeit einer Art der gepolsterten Fläche bestimmt sein. Ist die Fläche beispielsweise Bestandteil eines Stuhls oder eines Sessels, so kann die Position in einem Bereich liegen, auf dem üblicherweise Oberschenkel einer darauf sitzenden Person ruhen. Ist die Fläche Bestandteil eines Bettes, so kann die Position beispielsweise in einem Kopfbereich, einem Rumpfbereich oder einem Fußbereich einer im Bett liegenden Person liegen. Anderen gepolsterten Flächen können entsprechende Bereiche oder Positionen zugeordnet sein. So können Milben insbesondere dort bekämpft werden, wo sie mit gesteigerter Wahrscheinlichkeit oder in gesteigerter Anzahl auftreten. Die Bekämpfung der Milben kann so effizienter sein.

Es ist insbesondere bevorzugt, dass die Reinigungseinrichtung dazu eingerichtet ist, ein nichtthermisches Plasma bereitzustellen. Das Plasma kann derart bereitgestellt werden, dass Milben bekämpft und bevorzugt inaktiviert werden. Ein nichtthermisches Plasma (auch: kaltes atmosphärisches Plasma oder Niedertemperaturplasma, NTP) ist ein Plasma, das sich nicht im thermischen Gleichgewicht befindet, in welchem sich also z. B. die Temperaturen enthaltener Teilchensorten (Neutralteilchen, Ionen, Elektronen) signifikant unterscheiden. Ein kaltes atmosphärisches Plasma erzeugt eine reaktive Mischung aus Plasmakomponenten wie Elektronen, Ionen, angeregten Atomen und Molekülen, reaktiven Spezies (wie z.B. Os, NO, NO₂, etc.), UV-Strahlung und Wärme. Mittels dieser Mischung können Bakterien, Pilze, Viren, Sporen oder Geruchsmoleküle effektiv zerstört werden. Mittels NTP können bei einer Temperatur von unter 100° C zeitsparend Erreger sogar durch Kleidung oder Bettwäsche hindurch abgetötet werden. Auch kleine vielzellige Lebewesen wie Milben können mittels NTP abgetötet werden. Ein großer Vorteil des nichtthermischen Plasmas ist, dass es ungefähr Zimmertemperatur besitzt und bei Atmosphärendruck auf der Erde produziert werden kann. Zur Herstellung eines NTP kann ein Gas teilweise ironisiert werden, sodass nur ca. ein Teilchen aus 10⁹ ionisiert ist. Je nach verwendeter Plasmaquelle können Anteile umfasster Plasmakomponenten variieren oder auch gezielt verändert werden. So kann eine Konzentration oder Zusammensetzung von Plasmakomponenten an eine anvisierte Anwendung angepasst werden.

Das Plasma kann effizient gegen Milben eingesetzt werden, ohne eine Gesundheitsbelastung einer Person im Bereich der Fläche zu riskieren. Gleichzeitig kann mittels des Plasmas eine Desinfektion der Fläche erfolgen.

Es ist insbesondere bevorzugt, dass die Reinigungseinrichtung eine nichtthermische Plasmaquelle umfasst, die auf der Basis von Oberflächen-Mikroentladungen arbeitet. Eine solche Plasmaquelle ist beispielsweise in EP 3 326 436 A1 dargestellt. Derartige Plasmaquellen können mit einer relativ geringen elektrischen Spannung auskommen und energiesparend arbeiten, sodass sie sich für einen Batteriebetrieb eignen. Die Größe einer Elektrode, an der das Plasma bereitgestellt wird, kann in weiten Grenzen variiert werden. Die Elektrode selbst kann flach und flexibel ausgeführt werden. In einer weiteren Ausführungsform ist auch eine alternative Technologie zur Bereitstellung eines nichtthermischen Plasmas anwendbar, beispielsweise eine magnetisch organisierte Plasmaschichttechnologie, eine Mikrowellen-Plasmatechnologie oder eine Venturi-Plasmatechnologie.

Der Reinigungsroboter umfasst weiter bevorzugt ein Gebläse zur Absaugung von Luft aus dem Bereich der Fläche. Das Gebläse kann insbesondere von einem Saugwerk umfasst sein, wie es von einem Staubsaugroboter bekannt ist. Mittels des Gebläses können die Milben im Bereich der Fläche aufgenommen werden. Bevorzugt werden die aufgenommenen Milben, eventuell verbleibendes Lockmittel und/oder zusätzlich aufgenommene Schmutzpartikel aus der eingesogenen Luft abgeschieden und in einem Behälter aufgenommen. Die gepolsterte Fläche kann dadurch verbessert gereinigt werden.

Optional kann eine Einrichtung zum Lösen von Objekten von der Fläche vorgesehen sein, beispielsweise eine Bürste oder ein Besen. Die Einrichtung kann beweglich sein, um vom Reinigungsroboter gegenüber der Fläche bewegt zu werden. Schmutz, Partikel oder Milben können so verbessert von der Fläche gelöst werden. Insbesondere wenn die Fläche ein Textil umfasst, kann dieses durch die Einrichtung verbessert gereinigt werden. die Einrichtung kann oszillierend oder rotierend angetrieben werden und beispielsweise als Bürsten- oder Borstenwalze realisiert sein.

In einer weiteren Ausführungsform umfasst der Reinigungsroboter zusätzlich einen Filter für die abgesaugte Luft. Es können unterschiedliche Filter für unterschiedliche Zwecke vorgesehen sein. Beispielsweise kann ein erster Filter zur Rückhaltung von Staub, Schmutzpartikeln oder aufgenommenen Milben vorgesehen sein. Ein derartiger Filter kann insbesondere als Vliesfilter, Porenfilter oder Zyklonfilter ausgebildet sein. Dieser oder ein zweiter Filter kann zum Rückhalten von Ozon aus der abgesaugten Luft eingerichtet sein und beispielsweise einen Aktivkohlefilter umfassen. Ozon, das bei der Bereitstellung von kaltem Plasma an der Reinigungseinrichtung gebildet werden kann, kann so davon abgehalten werden, sich im Bereich der gepolsterten Fläche auszubreiten und von einer Person aufgenommen zu werden.

Der Reinigungsroboter ist bevorzugt zum autonomen Betrieb auf der gepolsterten Fläche eingerichtet. Dazu kann der Reinigungsroboter eine spezielle Antriebseinrichtung umfassen, um zu verhindern, dass er beispielsweise in einem sehr weichen Polster einsinkt und manövrierunfähig wird. Es wird vorgeschlagen, dass die Antriebseinrichtung wenigstens eines, bevorzugt aber zwei parallele Kettenlaufwerke umfasst. Das Kettenlaufwerk kann eine Gleiskette, ein Antriebsrad, ein Führungsrad und zwei oder mehr Laufrollen umfassen. Durch ihre große Auflagefläche auf der gepolsterten Fläche kann die Gleiskette nur einen geringen spezifischen Bodendruck bewirken, sodass der Reinigungsroboter auch auf sehr weichen oder glatten Flächen manövrierfähig bleiben kann.

Es ist weiter bevorzugt, dass der Reinigungsroboter wenigstens einen Sensor zur Bestimmung einer Begrenzung der Fläche aufweist. Dabei können unterschiedliche Begrenzungen unterschieden werden, insbesondere eine Stufe, die sich von der Fläche aus nach unten erstreckt, und eine Schwelle, die sich nach oben erstreckt. Eine Stufe kann beispielsweise am Ende einer Matratze gebildet sein. Eine Schwelle kann etwa durch ein Fußteil oder ein Haupt eines Bettes gebildet sein, in dem eine Matratze liegt, deren Oberfläche bearbeitet werden kann.

Nach einem zweiten Aspekt der vorliegenden Erfindung umfasst ein Verfahren zum Steuern eines Reinigungsroboters mit einer Reinigungseinrichtung über eine Fläche Schritte des Fahrens des Reinigungsroboters geradeaus bis zum Erreichen eines Hindernisses und des Bestimmens einer Hindernisposition; des Änderns der Fahrtrichtung um einen vorbestimmten Winkel und des Wiederholens des vorangehenden Schritts sooft, bis ausreichend viele Hindernispositionen bestimmt wurden, um Begrenzungen der Fläche zu bestimmen; und des Führens des Reinigungsroboters auf der Fläche in Abhängigkeit ihrer bestimmten Begrenzungen.

Das Verfahren eignet sich insbesondere, um mittels eines hierin beschriebenen Reinigungsroboter ausgeführt zu werden. Eine Steuereinrichtung des Reinigungsroboters kann eine Verarbeitungseinrichtung umfassen, die dazu eingerichtet ist, ein hierin beschriebenes Verfahren ganz oder teilweise auszuführen. Dazu kann die Verarbeitungseinrichtung einen programmierbaren Mikrocomputer oder Mikrocontroller umfassen und das Verfahren kann in Form eines Computerprogrammprodukts mit Programmcodemitteln vorliegen. Das Computerprogrammprodukt kann auch auf einem computerlesbaren Datenträger abgespeichert sein. Merkmale oder Vorteile des Verfahrens können auf die Steuereinrichtung bzw. den Reinigungsroboter übertragen werden und umgekehrt.

Es ist besonders bevorzugt, dass auf einem Bereich oder an einer Position auf der Fläche ein Lockmittel für Milben ausgegeben wird; und dass nach einer vorbestimmten Wartezeit nach dem Ausgeben eine Reinigungseinrichtung des Reinigungsroboters im Bereich des ausgegebenen Lockmittels aktiviert wird. Die Position oder der Bereich kann auf der Basis eines hierin beschriebenen Bestimmungsverfahrens für Begrenzungen der Fläche oder auf andere Weise bestimmt werden. Es ist weiter bevorzugt, dass der Reinigungsroboter während der Wartezeit von der Position weggefahren wird. Beispielsweise kann Lockmittel nacheinander an mehreren Positionen ausgegeben und später mittels der Reinigungseinrichtung wieder entfernt werden. Außerdem kann der Reinigungsroboter während der Wartezeit einen restlichen Bereich der zu reinigenden Fläche befahren und insbesondere mittels der Reinigungseinrichtung behandeln.

Es ist weiter bevorzugt, dass die Position, an der das Lockmittel ausgegeben wird, auf der Basis von Begrenzungen der Fläche bestimmt wird. Bezüglich der Begrenzungen kann ein Bereich vorgegeben sein, in dem die Position liegt. Der Bereich kann auch auf der Basis einer Art eines Möbelstücks bestimmt werden, von dem die gepolsterte Fläche umfasst ist. Beispielhafte Arten von Möbelstücken umfassen ein Bett, einen Sessel, einen Stuhl, einen Hocker und ein Sofa. Die Art des Möbelstücks kann auf der Basis von Begrenzungen der Fläche bestimmt werden. Beispielsweise kann eine halbrunde Fläche einer vorbestimmten Größe auf die Sitzfläche eines Sessels hinweisen, während eine langgestreckte, rechteckige Fläche einer anderen Größe auf die Sitzfläche einer Couch oder auf ein Bett hinweisen kann.

In einer weiter bevorzugten Ausführungsform ist die Fläche rechteckig, wobei der Winkel ca. 90° beträgt. Begrenzungen der Fläche können bereits in wenigen Zügen ausreichend genau bestimmt werden. Bevorzugt wird immer der gleiche Winkel verwendet; eine Verwendung unterschiedlicher Winkel ist jedoch ebenfalls vorstellbar. Der Winkel kann in einer Ausführungsform jeweils in einem Bereich zwischen ca. 45° und ca. 135° liegen. Insbesondere können vier Hindernispositionen bestimmt werden. Es wurde erkannt, dass bereits vier Hindernispositionen ausreichen können, um Begrenzungen der Fläche zu bestimmen. Zur Erhöhung einer Bestimmungssicherheit können auch mehr Hindernispositionen bestimmt werden.

Es ist weiterhin bevorzugt, dass eine Richtung bestimmt wird, in der sich ein Hindernis erstreckt; wobei die Begrenzungen zusätzlich auf der Basis einer Richtung eines Hindernisses bestimmt werden. Diese Vorgehensweise kann insbesondere an einer rechteckigen Fläche erfolgreich sein, wo das Hindernis eine Kante, eine Stufe oder eine Schwelle bilden kann.

Es ist weiterhin bevorzugt, dass unterschiedliche Arten von Hindernissen erkannt werden. Eine erste Art Hindernis kann erkannt werden, wenn der Reinigungsroboter an eine Schwelle in der Fläche fährt. Eine zweite Art Hindernis kann erkannt werden, wenn der Reinigungsroboter an eine Stufe in der Fläche fährt. Anhand der Art der Hindernisse kann eine Art eines Möbelstücks bestimmt werden, von dem die gepolsterte Fläche umfasst ist. Beispielsweise kann ein Bett eine rechteckige Polsterfläche umfassen, die an allen vier Seiten durch eine Schwelle oder eine Stufe begrenzt ist. Eine Couch kann eine ähnlich geformte Fläche aufweisen, ist jedoch üblicherweise an wenigstens einer Längsseite durch eine Schwelle begrenzt, beispielsweise in Form einer Rückenlehne oder einer Armlehne.

Nach einem weiteren Aspekt der vorliegenden Erfindung umfasst ein weiteres Verfahren zum Steuern eines Reinigungsroboters mit einer Reinigungseinrichtung Schritte des Bestimmens eines Bereichs der Fläche; des Ausbringens von Lockmittel auf dem Bereich; und des Bekämpfens von Milben in dem Bereich nach einer vorbestimmten Wartezeit.

Die Wartezeit liegt zwischen dem Ausbringen des Lockmittels und dem Bekämpfen der Milben. Das Bekämpfen kann ein Inaktivieren, optional ein Abtöten, sowie weiter optional ein Entfernen, insbesondere ein Absaugen umfassen, wie hierin genauer beschrieben ist. Das Inaktivieren oder Abtöten erfolgt bevorzugt mittels eines nichtthermischen Plasmas.

Der Reinigungsroboter kann nach dem Ausbringen von Lockmittel und vor dem Reinigen der Fläche eine vorbestimmte Bewegung ausführen, um Milben verbessert auf eine Inaktivierung und/oder Entfernung vorzubereiten.

Milben können eine begrenzte Mobilität aufweisen. Sie können von dem Lockmittel angelockt und/oder von Schall oder Vibrationen verschreckt werden. Der Reinigungsroboter kann die bei seiner Bewegung unvermeidlichen Vibrationen oder Geräusche dazu einsetzen, die Milben zu dem Bereich zu treiben, in dem das Lockmittel ausgebracht wurde, oder um sie in dem Bereich zu konzentrieren. Beispielsweise kann der Reinigungsroboter den Bereich umfahren, optional auch mehrfach und mit sinkendem Abstand, beispielsweise spiralförmig. Die Bewegung kann bis in den Bereich hinein fortgesetzt werden, um die Milben idealerweise an einer vorbestimmten Position im Bereich zu sammeln.

Der Reinigungsroboter kann nach dem Ausbringen des Lockmittels in dem Bereich ferner dazu gesteuert werden, einen umliegenden Bereich zu reinigen, insbesondere mittels Absaugen. Dadurch kann eine Feuchtigkeit im umliegenden Bereich reduziert werden, sodass der nicht abgesaugte Bereich für Milben attraktiver als der getrocknete umliegende Bereich ist. Eine Konzentration der Milben in dem mit Lockmittel versehenen Bereich kann dadurch weiter verbessert werden. Das Reinigen des umliegenden Bereichs kann zusammen mit dem systematischen Treiben von Milben in Richtung des mit Lockmittel versehenen Bereichs erfolgen.

Vor und/oder nach der vorbestimmten Bewegung kann der Reinigungsroboter während einer vorbestimmten Wartezeit in einen ruhigen Modus versetzt werden, in welchem er möglichst geringe oder gar keine Vibrationen und/oder Schall emittiert, um die Milben nicht aus dem Bereich zu verscheuchen. Schließlich können die Milben mittels der Reinigungsvorrichtung inaktiviert und/oder entfernt werden.

Die Erfindung wird nun unter Bezug auf die beiliegenden Figuren genauer beschrieben, in denen:
- Figur 1: einen Reinigungsroboter;
- Figur 2: ein Ablaufdiagramm eines Verfahrens zum Steuern eines Reinigungsroboters; und
- Figur 3: einen beispielhaften Pfad eines Reinigungsroboters über eine gepolsterte Fläche
darstellt.

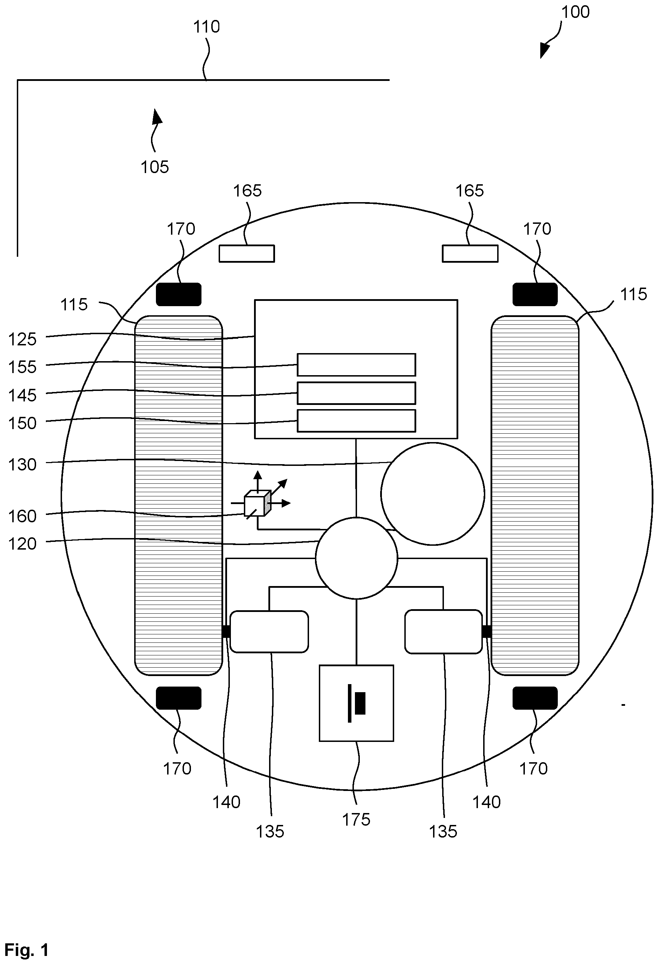

Figur 1 zeigt eine schematische Darstellung eines beispielhaften Reinigungsroboters 100, der dazu eingerichtet ist, eine Fläche 105 zu reinigen. Die Fläche 105 ist bevorzugt gepolstert und weiter bevorzugt von einem Möbelstück 110 umfasst. Das Möbelstück 110 kann dazu eingerichtet sein, eine Person insbesondere sitzend oder liegend aufzunehmen. Das Möbelstück 110 kann daher insbesondere ein Bett, ein Sofa, einen Sessel, einen Schemel oder Hocker oder einen Stuhl umfassen. In einer weiteren Ausführungsform kann die Fläche 105 auch im Wesentlichen flach auf dem Boden liegen, beispielsweise in Gestalt eines Teppichs. Der Reinigungsroboter 100 umfasst bevorzugt eine Antriebseinrichtung 115, eine Steuereinrichtung 120, eine Reinigungseinrichtung 125 und eine Ausgabevorrichtung 130.

Der Reinigungsroboter 100 ist beispielhaft rund nach der Art eines Diskus ausgebildet, kann jedoch auch anders geformt sein. Bevorzugt ist der Reinigungsroboter 100 derart geformt, dass er selbsttätig unter ein Kissen oder eine Decke eintauchen kann, die auf der Fläche 105 liegen können. Die Antriebseinrichtung 115 kann einen konventionellen Antrieb mit zwei oder mehr angetriebenen Rädern und optional einem oder mehreren gelenkten Rädern umfassen. In der dargestellten Ausführungsform umfasst die Antriebseinrichtung 115 zwei Kettenlaufwerke, die rechts und links einer Längsachse des Reinigungsroboters 100 angeordnet sind. Jedes Kettenlaufwerk 115 umfasst bevorzugt einen Antriebsmotor 135 und weiter bevorzugt einen Drehsensor 140 zur Bestimmung eines mit dem jeweiligen Kettenlaufwerk 115 zurückgelegten Wegs.

Die Reinigungseinrichtung 125 ist dazu eingerichtet, kleine Gegenstände von der Fläche 105 aufzunehmen und umfasst bevorzugt ein Gebläse 145, das von einem Saugwerk umfasst sein kann. Die Gegenstände können aufgenommen werden, indem Luft nahe an der Fläche 105 eingesogen wird, sodass die Gegenstände im Luftsog mitgerissen werden. Abgesaugte Luft kann in einem Filter 150 gefiltert werden. Ein erster Filter 150 kann mechanisch sein, um beispielsweise Staub, Milben oder Schmutzpartikel abzuscheiden. Ein zweiter Filter 150 kann chemisch ausgeführt sein, beispielsweise um Ozon abzuscheiden bzw. umzuwandeln. Der zweite Filter 150 ist bevorzugt in Form eines Aktivkohlefilters ausgeführt, dessen Kohlenstoff mit Ozon zu Kohlenstoffdioxid oxidiert werden kann. Das Ozon kann im Bereich der Fläche 105 gebildet werden, wenn eine von der Reinigungseinrichtung 125 umfasste Plasmaquelle 155 in Betrieb ist.

Die Plasmaquelle 155 ist bevorzugt dazu eingerichtet, ein nichtthermisches Plasma bereitzustellen und arbeitet weiter bevorzugt nach dem Prinzip der Oberflächen-Mikroentladungstechnologie. Anstelle der Plasmaquelle 155 kann auch eine andere Einrichtung zum Abtöten von Milben vorgesehen sein. Die Vorrichtung wird bevorzugt elektrisch betrieben und kann beispielsweise eine Quelle für ultraviolettes Licht umfassen. Die Ausgabevorrichtung 130 umfasst bevorzugt einen Vorratsbehälter für Lockmittel und eine Dosiereinrichtung zum Ausbringen einer steuerbaren Menge des Lockmittels auf die Fläche 105. Das Lockmittel ist bevorzugt pulverförmig, kann jedoch auch beispielsweise in flüssiger Form vorliegen. In einer Ausführungsform umfasst das Lockmittel Kieselgur.

Zur Kollisionsvermeidung, Orientierung oder Navigation können zusätzliche Sensoren vorgesehen sein. Ein Inertialsensor 160 kann einen Beschleunigungssensor und/oder einen Drehratensensor umfassen. Beide Arten von Sensoren arbeiten bevorzugt mehrdimensional und insbesondere in jeweils drei Raumrichtungen. Einer oder mehrere Nahfeldsensoren 165 können zur Abtastung eines Bereichs von ca. 0 bis 10 Zentimetern vor dem Reinigungsroboter 100 vorgesehen sein. Ein Nahfeldsensor 165 kann insbesondere dazu eingerichtet sein, eine Schwelle zu erkennen, also ein Hindernis, das von der Fläche 105 in vertikaler Richtung mehr als ein vorbestimmtes Maß aufragt. Das vorbestimmte Maß kann einige Zentimeter betragen, beispielsweise ca. 1 bis ca. 3 Zentimeter.

Ferner können einer oder mehrere Bodensensoren 170 vorgesehen sein, die jeweils dazu eingerichtet sind, eine Stufe unter dem Reinigungsroboter 100 zu erkennen. Eine Stufe erstreckt sich vertikal nach unten und hat üblicherweise eine bestimmte Mindesthöhe, beispielsweise einige Zentimeter oder Dezimeter. Eine Stufe kann auch mittels eines Neigungssensors bestimmt werden, der bestimmt, wenn sich der Reinigungsroboter 100 um mehr als ein vorbestimmtes Maß neigt, insbesondere in Fahrtrichtung. Zur Versorgung von Komponenten des Reinigungsroboters 100 mit Energie ist bevorzugt ein elektrischer Energiespeicher 175 vorgesehen. Damit kann der Reinigungsroboter 100 unabhängig von einem Energienetz betrieben werden.

Der Reinigungsroboter 100 kann über weitere Komponenten verfügen, die an einem Gerät des Standes der Technik bekannt sind, etwa eine Kommunikationseinrichtung oder eine Ausgabevorrichtung 130 in beispielsweise optischer, haptischer oder akustischer Weise. Zusätzliche Sensoren können einen oder mehrere Fernbereichssensoren umfassen, die insbesondere als Kamera, Radarsensor, LiDAR-Sensor oder Tiefenkamera ausgebildet sein können. Zum Betrieb einer Kamera kann außerdem eine Beleuchtungseinrichtung vorgesehen sein.

Es wird vorgeschlagen, dass der Reinigungsroboter 100 dazu eingerichtet ist, die Fläche 105 autonom von Milben zu reinigen. Dazu kann der Reinigungsroboter 100 zunächst bestimmte Eigenschaften der Fläche 105 bestimmen, um dann mittels der Ausgabevorrichtung 130 ein Lockmittel für Milben an einer vorbestimmten Position auf der Fläche 105 auszubringen und nach einer vorbestimmten Zeit mittels der Reinigungseinrichtung 125 wieder aufzunehmen. Vor oder nach dem Aufnehmen können die Milben mittels der Plasmaquelle 155 abgetötet werden.

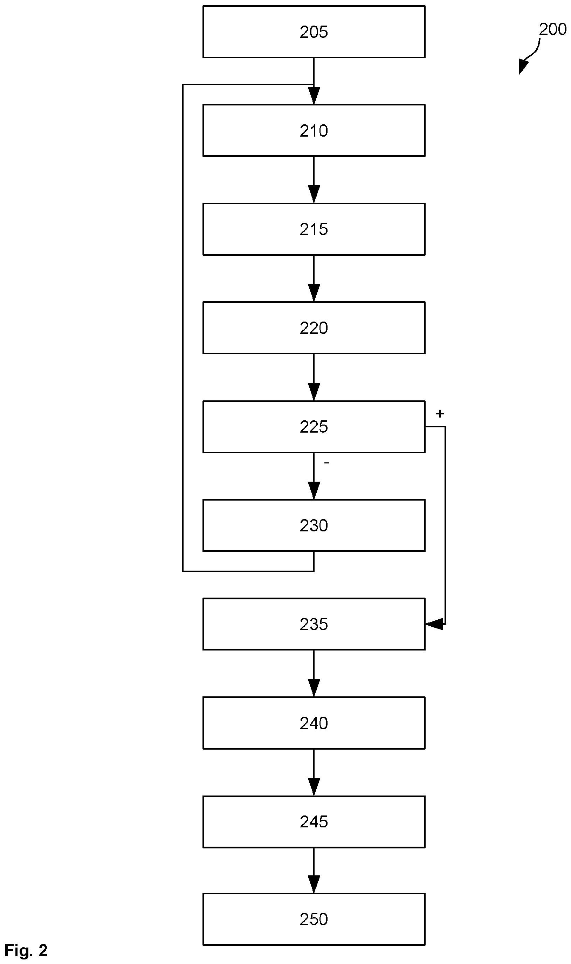

Figur 2 zeigt ein Ablaufdiagramm eines beispielhaften Verfahrens 200 zur Steuerung eines Reinigungsroboters 100. In einem ersten Schritt 205 befindet sich der Reinigungsroboter 100 an einer Startposition auf der Fläche 105. Die Startposition kann beliebig gewählt sein und der Reinigungsroboter 100 kann beispielsweise durch eine Bedienperson an der Startposition auf die Fläche 105 gelegt und aktiviert werden.

In einem Schritt 210 kann der Reinigungsroboter 100 in einer beliebigen Richtung auf der Fläche 105 geradeaus fahren, bis er in einem Schritt 215 ein Hindernis erfasst, das seine Weiterfahrt stört. Der Reinigungsroboter 100 kann angehalten werden und in einem Schritt 220 kann eine Hindernisposition bestimmt werden, an der sich der Reinigungsroboter 100 bzw. das erfasste Hindernis befindet. Es ist bevorzugt, dass zusätzlich eine Richtung bestimmt wird, in der sich das Hindernis bezüglich des Reinigungsroboters 100 erstreckt.

In einem Schritt 225 kann überprüft werden, ob ausreichend Hindernispositionen bestimmt wurden, um Begrenzungen der Fläche 105 mit einer vorbestimmten Genauigkeit oder Zuverlässigkeit zu bestimmen. Ist dies nicht der Fall, so kann der Reinigungsroboter 100 in einem Schritt 230 seine Richtung um einen vorbestimmten Winkel ändern, der insbesondere 90° betragen kann, und das Verfahren 200 kann im Schritt 210 fortfahren, sodass die Schritte 210 bis 225 erneut durchlaufen werden.

Insbesondere falls im Schritt 225 bestimmt wurde, dass ausreichend Hindernispositionen vorliegen, können in einem Schritt 235 Begrenzungen der Fläche 105 bestimmt werden. Die Begrenzungen können beispielsweise gebildet werden, indem zuvor bestimmte Hindernispositionen miteinander verbunden werden. Dabei kann eine vorbestimmte Form der Fläche 105 bestimmt oder vorausgesetzt werden. In einer Ausführungsform kann die Form auf der Basis der bestimmten Hindernispositionen bestimmt werden, bevor die Begrenzungen der Fläche 105 bestimmt werden. Unterschiedliche Formen können für unterschiedliche Möbelstücke 110 typisch sein, von denen eine zu reinigende Fläche 105 umfasst sein kann. Eine Art eines vorliegenden Möbelstücks 110 kann beispielsweise in Form einer externen Vorgabe vorliegen oder auf der Basis bestimmter Hindernispositionen bzw. einer bestimmten Form von Begrenzungen der Fläche 105 seitens des Reinigungsroboters 100 bestimmt werden.

In einem Schritt 240 kann der Reinigungsroboter 100 an eine vorbestimmte Position bewegt werden, wobei die Position in Abhängigkeit der bestimmten Form, des bestimmten Möbelstücks 110 oder einer externen Vorgabe definiert sein kann. Anschließend kann mittels der Ausgabevorrichtung 130 Lockmittel für Milben auf die Fläche 105 ausgegeben werden.

In einem Schritt 245 vergeht bevorzugt mindestens eine vorbestimmte Zeit, in der die Milben durch das ausgegebene Lockmittel angelockt werden. In dieser Zeit kann der Reinigungsroboter 100 beispielsweise eine weitere Position auf der Fläche 105 anfahren und dort weiteres Lockmittel ausgeben. Außerdem kann er mittels der Reinigungseinrichtung 125 einen Bereich der Fläche 105 bearbeiten, an dem kein Lockmittel ausgegeben wurde.

Nach Ablauf der vorbestimmten Zeit kann der Reinigungsroboter 100 in einem Schritt 250 zu der Position vom Schritt 240 zurückkehren und Milben oder andere Fremdkörper im Bereich der Position aufnehmen. Dazu kann er insbesondere das Gebläse 145 verwenden, um die Fläche 105 in diesem Bereich abzusaugen. Überschüssiges Lockmittel und andere lose Partikel wie Staub oder Schmutzteilchen können dabei ebenfalls eingesaugt werden. Wurde Lockmittel an unterschiedlichen Positionen auf der Fläche 105 ausgebracht, so kann die Fläche 105 im Bereich aller Positionen mittels des Gebläses 145 gereinigt werden.

In einer anderen Ausführungsform kann auch die gesamte Fläche 105 gereinigt werden, um die Milben, überschüssiges Lockmittel und andere Partikel an der oder den Positionen aufzunehmen.

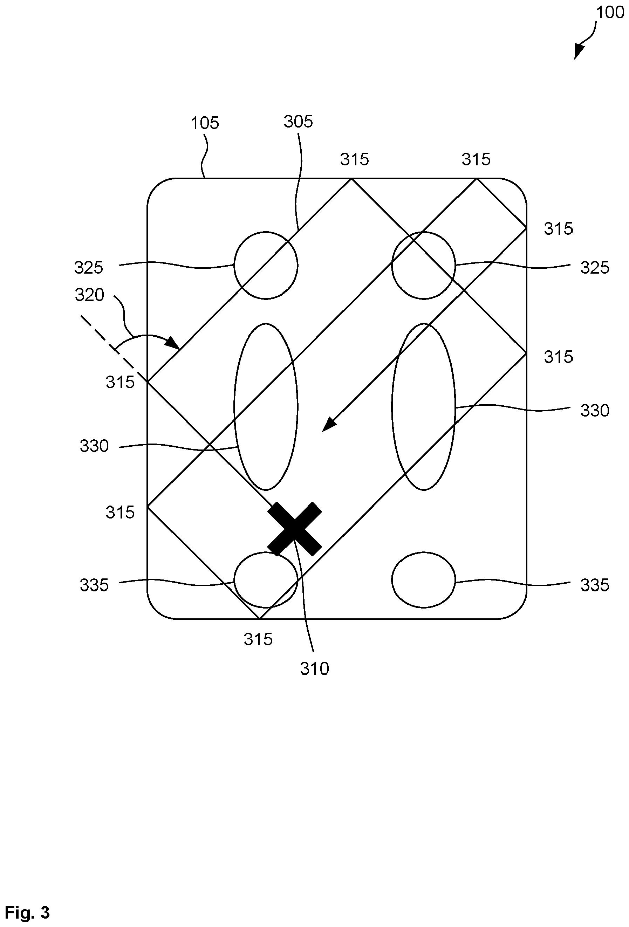

Figur 3 zeigt einen beispielhaften Pfad 305 eines Reinigungsroboters 100 über eine gepolsterte Fläche 105. Beispielhaft sei die Fläche 105 eine Oberfläche eines Möbelstücks 110 angenommen, das als Doppelbett genutzt werden kann.

Der Pfad 305 beginnt an einer Startposition 310, die zufällig gewählt sein kann. Auch eine Ausrichtung des Reinigungsroboters 100 an der Startposition 310 kann zufällig sein. Von dort verläuft der Pfad 305 in der gewählten Richtung jeweils geradeaus, bis eine Hindernisposition 315 erfasst wurde, die auf eine Begrenzung der Fläche 105 hinweist. Der Reinigungsroboter 100 ändert dort seinen Kurs um einen vorbestimmten Winkel 320, der vorliegend beispielhaft 90° im Uhrzeigersinn beträgt. Verläuft ein Hindernis an der Hindernisposition 315 in einer Richtung, so kann der Kurs so geändert werden, dass er in eine vom Hindernis entfernte Richtung weist. Anschließend kann der Reinigungsroboter 100 wieder geradeaus fahren, bis er an eine weitere Hindernisposition 315 gelangt.

Wurden ausreichend Hindernispositionen 315 erfasst, so können Begrenzungen der Fläche 105 bestimmt werden. Insbesondere wenn eine Anzahl von erfassten Hindernispositionen 315 ausreichend groß ist oder wenn zwischen benachbarten Hindernispositionen 315 jeweils ein kleinerer als ein vorbestimmter Abstand besteht, können die Begrenzungen der Fläche 105 mit ausreichender Sicherheit oder Genauigkeit bestimmt werden. Sind die Hindernispositionen 315 sehr unterschiedlich voneinander beabstandet, so kann eine vermutete Hindernisposition 315 gezielt gesucht werden, beispielsweise in einem Bereich zwischen bestimmten Hindernispositionen 315.

Aufgrund ihrer Größe, ihrer Form oder eines Verhältnisses von Seitenlängen kann die Fläche 105 als Oberfläche eines Doppelbetts erkannt werden. Dieser Fläche 105 können einer oder mehrere Bereiche zugeordnet sein, an denen mit vermehrtem Aufkommen von Milben zu rechnen sein kann. Im gewählten Beispiel können dies zwei Kopfbereiche 325, zwei Rumpfbereiche 330 und/oder drei Fußbereiche 335 umfassen. Die Bereiche 325 bis 335 können danach bestimmt sein, wo sich bestimmte Abschnitte einer Person üblicherweise befinden, wenn diese das Möbelstück 110 benutzt, das die Fläche 105 umfasst. In einem oder mehreren der Bereiche 325 bis 335 kann der Reinigungsroboter 100 anschließend mit der Bekämpfung von Milben beginnen. Dazu kann insbesondere Lockmittel für Milben an einer Position in einem der Bereiche 325 bis 335 oder auf einem Abschnitt eines der Bereiche 325 bis 335 ausgebracht und später wieder abgesaugt werden.

In einer weiteren Ausführungsform kann ein Bereich, in dem Lockmittel ausgebracht wird, auf der Basis anderer oder zusätzlicher Kriterien bestimmt werden. Der Bereich kann so bestimmt werden, dass er einen vorbestimmten Abstand zu einem Hindernis, insbesondere einer Begrenzung der Fläche 105, einhält. Ferner kann eine Form des Bereichs so gewählt werden, dass der Bereich mit einfachen Fahrmanövern durch den Reinigungsroboter 100 möglichst leicht vollständig überstrichen werden kann. Ein einfaches Fahrmanöver kann ein Geradeausfahren vorwärts oder rückwärts umfassen. Ein zeitraubendes Drehen des Reinigungsroboters 100 um seine Hochachse kann minimiert sein. So kann der Bereich zügig gereinigt werden, bevor sich angelockte Milben durch Schall oder Vibrationen des Reinigungsroboters 100 wieder zurückziehen können. Außerdem kann der Bereich so gewählt sein, dass ein möglichst großer Teil von ihm leicht überfahren werden kann, also bevorzugt keine Hindernisse aufweist und weiter bevorzugt zügig befahrbar ist. Der Bereich kann so gewählt sein, dass eine Oberfläche der Fläche 105 im Bereich möglichst gut zum Ausbringen von Lockmittel und/oder zum Reinigen geeignet ist. Dazu kann die Fläche 105 im Bereich möglichst eben sein und möglichst keine Ritzen oder Falten aufweisen.

Ein Reinigen der Fläche 105 nach der Wartezeit kann die ganze Fläche 105 oder nur einen Teil davon betreffen. Wurde nur in einem vorbestimmten Bereich Lockmittel ausgebracht, so kann die Reinigung nur in diesem Bereich oder in einem nur geringfügig überlappenden Bereich erfolgen.

Es ist zu beachten, dass das Verfahren 200 nicht notwendigerweise alle hierin genannten Schritte umfassen muss. In einer Ausführungsform können insbesondere die Schritte 210 bis 230 entfallen oder durch eine andere Heuristik ersetzt sein. Die Begrenzungen der Fläche 105 können im Schritt 235 beispielsweise mittels einer Navigationseinrichtung, auf der Basis einer Umgebungskarte oder aufgrund einer zurückliegenden Befahrung der Fläche 105 bereits bekannt sein.

Zwischen dem Ausgeben von Lockmittel im Schritt 240 und dem Aufnehmen von Milben im Schritt 250 kann der Reinigungsroboter auf eine vorbestimmte Weise über die Fläche 105 bewegt werden, um eine Konzentration von Milben in dem mit Lockmittel versehenen Bereich zu verbessern.

### Bezugszeichen

- 100: Reinigungsroboter
- 105: Fläche
- 110: Möbelstück
- 115: Antriebseinrichtung (Kettenlaufwerk)
- 120: Steuereinrichtung
- 125: Reinigungseinrichtung
- 130: Ausgabevorrichtung
- 135: Antriebsmotor
- 140: Drehsensor
- 145: Gebläse
- 150: Filter
- 155: Plasmaquelle
- 160: Inertialsensor
- 165: Nahfeldsensor
- 170: Bodensensor
- 175: Energiespeicher

- 200: Verfahren
- 205: Reinigungsroboter an Startposition
- 210: geradeaus fahren
- 215: Hindernis erfassen
- 220: Hindernisposition bestimmen
- 225: ausreichend Hindernispositionen bestimmt?
- 230: Richtung ändern
- 235: Begrenzungen bestimmen
- 240: Position bestimmen und Lockmittel an der Position ausgeben
- 245: vorbestimmte Zeit abwarten
- 250: Milben an Position aufnehmen

- 305: Pfad
- 310: Startposition
- 315: Hindernisposition
- 320: Winkel
- 325: Kopfbereich
- 330: Rumpfbereich
- 335: Fußbereich

## Patentansprüche

1. Reinigungsroboter (100) für eine gepolsterte Fläche (105), wobei der Reinigungsroboter (100) folgendes umfasst:
- eine Antriebseinrichtung (115);
- eine Steuereinrichtung (120) zur Führung des Reinigungsroboters (100) über die zu reinigende Fläche (105);
- eine Reinigungseinrichtung (125), die dazu eingerichtet ist, Milben im Bereich der Fläche (105) zu bekämpfen; **dadurch gekennzeichnet, dass** der Reinigungsroboter folgendes umfasst:
- eine Ausgabevorrichtung (130) für ein Lockmittel für Milben;
- wobei die Steuereinrichtung (120) dazu eingerichtet ist, Lockmittel auf der Fläche (105) auszugeben und nach einer vorbestimmten Wartezeit die Reinigungseinrichtung (125) im Bereich des ausgegebenen Lockmittels zu aktivieren.

2. Reinigungsroboter (100) nach Anspruch 1, wobei das Bekämpfen ein Inaktivieren umfasst.

3. Reinigungsroboter (100) nach Anspruch 2, wobei das Inaktivieren ein Abtöten umfasst.

4. Reinigungsroboter (100) nach einem der vorangehenden Ansprüche, wobei die Reinigungseinrichtung (125) dazu eingerichtet ist, Milben von der Fläche (105) zu entfernen.

5. Reinigungsroboter (100) nach einem der vorangehenden Ansprüche, wobei das Lockmittel dazu eingerichtet ist, die Milben nicht zu schädigen.

6. Reinigungsroboter (100) nach einem der vorangehenden Ansprüche, wobei die Steuereinrichtung (120) dazu eingerichtet ist, eine Emission von Schall oder Vibrationen durch den Reinigungsroboter (100) während der Wartezeit zu minimieren.

7. Reinigungsroboter (100) nach einem der vorangehenden Ansprüche, wobei die Steuereinrichtung (120) dazu eingerichtet ist, das Lockmittel an einer vorbestimmten Stelle der gepolsterten Fläche (105) auszugeben.

8. Reinigungsroboter (100) nach einem der vorangehenden Ansprüche, wobei die Reinigungseinrichtung (125) dazu eingerichtet ist, ein nichtthermisches Plasma bereitzustellen.

9. Reinigungsroboter (100) nach einem der vorangehenden Ansprüche, ferner umfassend ein Gebläse (145) zur Absaugung von Luft aus dem Bereich der Fläche (105).

10. Reinigungsroboter (100) nach Anspruch 9, ferner umfassend einen Filter (150) für die abgesaugte Luft.

11. Reinigungsroboter (100) nach einem der vorangehenden Ansprüche, wobei die Antriebseinrichtung (115) wenigstens ein Kettenlaufwerk umfasst.

12. Reinigungsroboter (100) nach einem der vorangehenden Ansprüche, ferner umfassend einen Sensor zur Bestimmung einer Begrenzung der Fläche (105).

13. Verfahren (200) zum Steuern eines Reinigungsroboters (100) mit einer Reinigungseinrichtung (125), wobei das Verfahren (200) folgende Schritte umfasst:
- Bestimmen (235) eines Bereichs der Fläche (105);
- Ausbringen (240) von Lockmittel auf dem Bereich; und
- Bekämpfen (250) von Milben in dem Bereich mit der Reinigungsvorrichtung (125) nach einer vorbestimmten Wartezeit.

## Claims

1. Cleaning robot (100) for a cushioned surface (105), wherein the cleaning robot (100) comprises the following:
- a drive facility (115);
- a control facility (120) for guiding the cleaning robot (100) over the surface (105) to be cleaned;
- a cleaning facility (125), which is designed to fight dust mites in the region of the surface (105); **characterised in that** the cleaning robot comprises the following:
- an output apparatus (130) for a lure for dust mites;
- wherein the control facility (120) is designed to output lures onto the surface (105) and after a predetermined waiting time to activate the cleaning facility (125) in the region of the output lure.

2. Cleaning robot (100) according to claim 1, wherein fighting of dust mites involves inactivation.

3. Cleaning robot (100) according to claim 2, wherein the inactivation involves killing.

4. Cleaning robot (100) according to one of the preceding claims, wherein the cleaning facility (125) is designed to remove dust mites from the surface (105).

5. Cleaning robot (100) according to one of the preceding claims, wherein the lure is designed not to harm the dust mites.

6. Cleaning robot (100) according to one of the preceding claims, wherein the control facility (120) is designed to minimise an emission of sound or vibrations by the cleaning robot (100) during the waiting time.

7. Cleaning robot (100) according to one of the preceding claims, wherein the control facility (120) is designed to output the lure at a predetermined point on the cushioned surface (105).

8. Cleaning robot (100) according to one of the preceding claims, wherein the cleaning facility (125) is designed to provide a non-thermal plasma.

9. Cleaning robot (100) according to one of the preceding claims, further comprising a fan (145) for extracting air from the region of the surface (105).

10. Cleaning robot (100) according to claim 9, further comprising a filter (150) for the extracted air.

11. Cleaning robot (100) according to one of the preceding claims, wherein the drive facility (115) comprises at least one tracked running gear.

12. Cleaning robot (100) according to one of the preceding claims, further comprising a sensor for determining a boundary of the surface (105).

13. Method (200) for controlling a cleaning robot (100) with a cleaning facility (125), wherein the method (200) comprises the following steps:
- determining (235) a region of the surface (105);
- applying (240) lures to the region; and
- fighting (250) dust mites in the region with the cleaning apparatus (125) after a predetermined waiting time.

## Revendications

1. Robot de nettoyage (100) pour une surface rembourrée (105), dans lequel le robot de nettoyage (100) comprend:
- un dispositif d'entraînement (115) ;
- un dispositif de commande (120) destiné au guidage du robot de nettoyage (100) au-dessus de la surface (105) à nettoyer ;
- un dispositif de nettoyage (125) qui est conçu pour lutter contre des acariens dans la zone de la surface (105) ; **caractérisé en ce que** le robot de nettoyage comprend :
- un dispositif de distribution (130) pour un leurre pour des acariens ;
- dans lequel le dispositif de commande (120) est conçu pour distribuer des leurres sur la surface (105) et activer le dispositif de nettoyage (125) dans la zone du leurre distribué au terme d'un temps d'attente prédéterminé.

2. Robot de nettoyage (100) selon la revendication 1, dans lequel la lutte comprend une inactivation.

3. Robot de nettoyage (100) selon la revendication 2, dans lequel l'inactivation comprend une élimination.

4. Robot de nettoyage (100) selon l'une quelconque des revendications précédentes, dans lequel le dispositif de nettoyage (125) est conçu pour enlever des acariens de la surface (105).

5. Robot de nettoyage (100) selon l'une quelconque des revendications précédentes, dans lequel le leurre est conçu pour ne pas nuire aux acariens.

6. Robot de nettoyage (100) selon l'une quelconque des revendications précédentes, dans lequel le dispositif de commande (120) est conçu pour minimiser une émission de bruit ou de vibrations par le robot de nettoyage (100) pendant le temps d'attente.

7. Robot de nettoyage (100) selon l'une quelconque des revendications précédentes, dans lequel le dispositif de commande (120) est conçu pour distribuer le leurre au niveau d'un point prédéterminé de la surface rembourrée (105).

8. Robot de nettoyage (100) selon l'une quelconque des revendications précédentes, dans lequel le dispositif de nettoyage (125) est conçu pour fournir un plasma non thermique.

9. Robot de nettoyage (100) selon l'une quelconque des revendications précédentes, comprenant en outre une soufflante (145) destinée à l'aspiration d'air à partir de la zone de la surface (105).

10. Robot de nettoyage (100) selon la revendication 9, comprenant en outre un filtre (150) pour l'air aspiré.

11. Robot de nettoyage (100) selon l'une quelconque des revendications précédentes, dans lequel le dispositif d'entraînement (115) comprend au moins un train de roulement à chenilles.

12. Robot de nettoyage (100) selon l'une quelconque des revendications précédentes, comprenant en outre un capteur destiné à la détermination d'une limitation de la surface (105).

13. Procédé (200) destiné à la commande d'un robot de nettoyage (100) avec un dispositif de nettoyage (125), dans lequel le procédé (200) comprend les étapes suivantes :
- détermination (235) d'une zone de la surface (105) ;
- extraction (240) de leurre sur la zone ; et
- lutte (250) contre des acariens dans la zone avec le dispositif de nettoyage (125) au terme d'un temps d'attente prédéterminé.
